# FASCICULE DE BREVET EUROPEEN

(11) **EP 2 136 710 B1**
(45) Date de publication et mention de la délivrance du brevet: **29.08.2012**
(21) Numéro de dépôt: 08736223.2
(22) Date de dépôt: 15.04.2008
(51) Int. Cl.: A61B 6/14

(54) **CAPTEUR D'IMAGE RADIOLOGIQUE DENTAIRE AVEC SURMOULAGE SOUPLE**
BILDSENSOR FÜR ZAHNRÖNTGEN MIT FLEXIBLER ÜBERSPRITZUNG
DENTAL RADIOLOGY IMAGE SENSOR WITH FLEXIBLE OVERMOULDING

(30) Priorité: 20.04.2007 FR 0702891
(43) Date de publication de la demande: 30.12.2009
(73) Titulaire: E2V Semiconductors, 38120 Saint Egrève (FR)
(72) Inventeur: GUICHARD, Jérôme, F-38470 Vatilieu (FR); JULIEN, Florian, F-38500 Voiron (FR)
(74) Mandataire: Guérin, Michel
(86) Numéro de dépôt international: PCT/EP2008/054529
(87) Numéro de publication internationale: WO 2008/135348

(56) Documents cités:
- EP-A- 1 661 518
- EP-A- 1 699 232
- WO-A-00/29872
- US-A- 6 030 119
- US-A1- 2006 067 462
- US-A1- 2007 025 523

## Description

L'invention concerne un capteur radiologique dentaire intra-oral.

Les capteurs intra-oraux dentaires actuels souffrent de plusieurs inconvénients que les fabricants aimeraient circonvenir.

Ils sont souvent inconfortables pour le patient en raison des angles vifs du boîtier qui tend à épouser la forme rectangulaire du capteur en silicium qu'il contient. Le matériau qui constitue le boîtier est dur, pour des raisons impératives de résistance mécanique, et peu agréable en termes de sensation dans la bouche. La dureté du matériau exige en pratique que les circuits électroniques internes au boîtier soient protégés des chocs (en cas de chute par exemple) par une feuille de mousse ou d'élastomère interposée entre les circuits électroniques et la paroi du boîtier, voire même par des feuilles d'autres matériaux ayant des fonctions supplémentaires (feuille d'aluminium, feuille de plomb). US 2006/0067462 divulgue des barettes élastiques de protection, interposées entre le module électronique et le boitier du capteur dentaire.

Les capteurs sont le plus souvent de couleur noire, ce qui est désagréable à l'oeil de l'utilisateur, ou même du médecin ; dans ce domaine on attendrait plutôt du blanc, couleur usuelle des appareils médicaux, symbolique de propreté. Mais si on les faisait blancs, ou même colorés, le risque de produire une image non désirée du fait de la lumière ambiante serait important. En effet, le boîtier est nécessairement de faible épaisseur pour des raisons d'encombrement et est dès lors relativement transparent ; et l'éclairage chez un praticien dentaire est souvent très puissant. On verrait alors, en l'absence d'illumination X, une image non noire apparaître sur l'écran qui est installé devant le praticien dentiste ou même devant le patient ; une telle image sans signification est intempestive et ne devrait pas apparaître pour ne pas gêner le patient ou le praticien.

Puisque les boîtiers sont tous noirs et sont géométriquement très semblables, les différents fabricants ne peuvent pas facilement différencier leurs produits de ceux des fabricants concurrents. Mais les fabricants, et d'ailleurs les acheteurs aussi, souhaitent une différentiation entre leurs produits et ceux de leurs concurrents, différentiation qui est difficile si tous les capteurs sont noirs, de même forme et de même dimensions.

US-A-6 030 119 décrit une housse amovible de protection en matière plastique souple pour protéger un capteur d'imagerie dentaire.

Plus généralement, le marché de ces capteurs est demandeur d'améliorations en termes de confort et d'ergonomie.

La présente invention a pour objet la prise en compte de ces multiples contraintes et la réalisation d'un capteur d'image radiologique dentaire intra-oral qui, sans altérer les fonctionnalités de prise d'image, soit plus agréable à la bouche, plus agréable à l'oeil, en même temps que distinctif, solide, et protégé au mieux contre les chocs.

Pour y parvenir, on propose selon l'invention un capteur d'image radiologique dentaire intra-oral comportant un module électronique de prise d'image et un boîtier moulé en matière plastique dure, caractérisé en ce que le boîtier comporte localement des surmoulages en une matière plastique souple à consistance de gomme lisse, au moins sur les angles les plus vifs de la matière plastique dure à l'extérieur du boîtier ainsi que sur des zones internes de la matière plastique dure, en des endroits où le module électronique peut venir s'appuyer, les parties surmoulées en matière souple à l'intérieur du boîtier étant reliées à des parties en matière souple à l'extérieur du boîtier par des ponts formés dans des ouvertures ménagées dans les parties en matière dure.

La forme de la partie en matière plastique dure et la forme de la matière plastique souple surmoulée sur la matière dure sont définies de telle sorte que l'extérieur du boîtier soit dépourvue d'aspérités, notamment à la transition entre zones de matière plastique souple surmoulée et zones de matière plastique dure dépourvue de surmoulage.

La matière plastique dure peut être une matière semi-cristalline tel que du polyamide. La matière plus souple peut être un copolymère de type SEBS (Styrène-Ethylène-Butylène-Styrène).

Ces matières peuvent être facilement surmoulées ensemble par injection. La matière plus souple est de préférence colorée en blanc ou en couleur. Elle peut être imprimée d'un dessin ou de caractères.

Dans une réalisation particulière, le boîtier comporte un dôme saillant pour une sortie de câble électrique et ce dôme est constitué uniquement par la matière plastique souple.

Dans une autre réalisation, une partie de la coque est spécifiquement destinée à former une zone sensible au toucher pour le doigt du praticien afin de l'aider à positionner le capteur dans la bouche du patient. Cette zone sensible spécifique est revêtue de la matière plastique souple à consistance de gomme lisse.

D'autres caractéristiques et avantages de l'invention apparaîtront à la lecture de la description détaillée qui suit et qui est faite en référence aux dessins annexés dans lesquels :
- la figure 1 représente une vue générale de principe, en coupe longitudinale, d'un capteur d'image radiologique dentaire intraoral en boîtier de matière plastique dure ;
- la figure 2 représente une coupe transversale du boîtier de la figure 1 ;
- la figure 3 représente une vue en perspective coupée, de dessus, d'un boîtier de capteur d'image radiologique dentaire intraoral selon l'invention ;
- la figure 4 représente une vue de dessous en perspective du boîtier de la figure 3 ;
- la figure 5 représente une vue en perspective de dessus, non coupée ;
- la figure 6 représente, en coupe, une variante de réalisation avec dôme souple ;
- et la figure 7 représente en vue de dessus en perspective la réalisation de la figure 6.

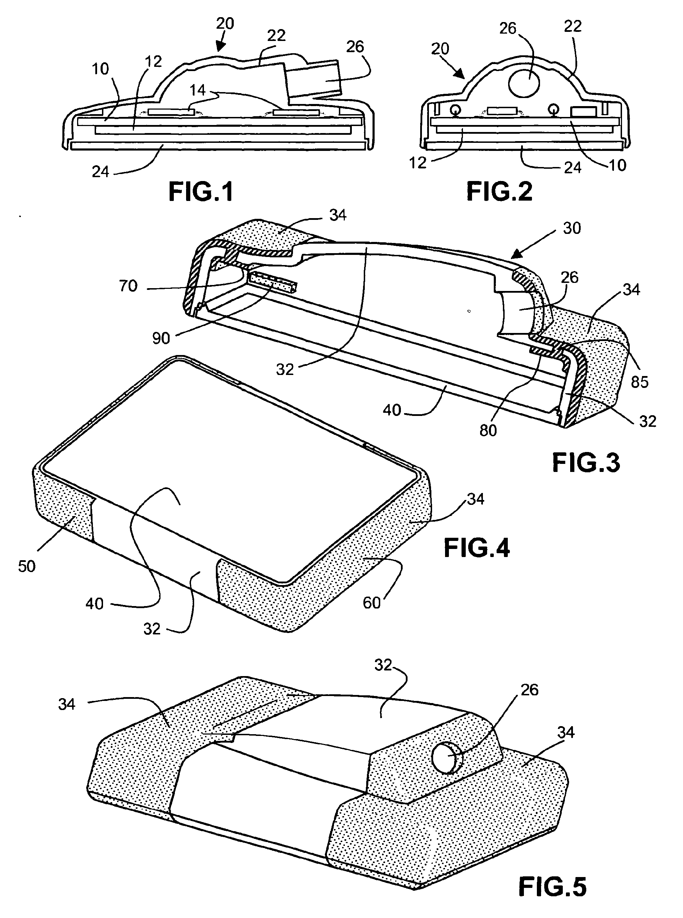

Sur les figures 1 et 2, on voit la constitution de principe classique d'un capteur radiologique dentaire intraoral. Ses dimensions latérales sont de quelques centimètres en longueur et en largeur, et de l'ordre du centimètre en hauteur, pour pouvoir être placé dans la bouche d'un patient.

Le capteur comporte une carte de circuit électronique 10 qui porte une puce de circuit intégré constituant le capteur d'image matriciel 12 proprement dit, et divers composants discrets 14. Dans cet exemple, la puce de circuit intégré est montée à l'arrière de la carte 10 et les composants discrets sont à l'avant.

La carte est enfermée dans un boîtier étanche 20 qui comprend une coque 22 et une plaque de fond 24 qui est soudée à la coque après montage de la carte dans le capot. La coque est pourvue d'une ouverture circulaire 26 permettant le passage d'un câble électrique multiconducteurs assurant la liaison entre la carte -- à l'intérieur du boîtier -- et l'extérieur. Les conducteurs du câble sont soudés à la carte électronique ou soudés à un connecteur pouvant s'enficher dans un connecteur complémentaire fixé à la carte. Le câble, non représenté, est pourvu d'une gaine isolante étanche dont le diamètre épouse le contour de l'ouverture circulaire 26 de manière à assurer l'étanchéité entre l'extérieur et l'intérieur du boîtier.

Dans la technique antérieure, la coque 22 et la plaque de fond 24 du boîtier sont en matière plastique dure et des plaquettes de mousse antichoc (non représentées) peuvent être disposées notamment entre le boîtier et la puce de circuit intégré. Pour la protection contre les chocs, on pouvait aussi prévoir d'enrober la carte avec des feuilles d'aluminium et de plomb, assurant également un rôle de protection contre les perturbations électromagnétiques. L'ensemble de ces éléments occupait un volume important.

Le principe du boîtier selon l'invention est représenté aux figures 3 à 5. Le boîtier est vide : la carte électronique n'est pas représentée à l'intérieur.

La coque est désignée par 30 et la plaque de fond par 40. La coque est constituée de deux matériaux différents soudés ensemble. Le premier matériau 32 est une matière plastique dure analogue à celle qui était utilisée auparavant. Il est de préférence noir pour être aussi peu transparent que possible à la lumière. Ce matériau peut être du polyamide. Le deuxième matériau 34 est une matière plastique souple à consistance de gomme lisse, tel qu'un copolymère SEBS (Styrène, Ethylène, Butylène, Styrène).

La surface du deuxième matériau est indiquée par des pointillés sur les figures ; les coupes de ce matériau sont représentées par des hachures. La surface et les coupes du premier matériau sont en blanc.

La plaque de fond 40 qui ferme la coque est de préférence faite uniquement dans le premier matériau rigide. Elle est soudée par ultrasons ou collée sur le rebord de fond de la coque, ce rebord étant réalisé dans le premier matériau rigide 32.

Le matériau 34 à consistance plus souple est surmoulé sur le premier matériau et possède des formes arrondies pour faire disparaître les angles vifs du premier matériau 32. Il n'est cependant pas surmoulé sur toute la surface extérieure du premier matériau, comme on le voit notamment sur les figures 4 et 5. Certaines parties du premier matériau restent visibles, non recouvertes par le second. Dans l'exemple représenté, il y a deux zones 50 et 60 de deuxième matériau 34, l'une du côté de la sortie de câble (ouverture 26), l'autre du côté opposé. Ces zones entourent complètement les coins du parallélépipède rectangle que forme globalement la coque 30. Leur forme est arrondie autour de ces coins.

La rigidité de la coque est principalement assurée par le premier matériau plus dur. Le deuxième matériau est plaqué contre le premier lors d'une opération de surmoulage. La configuration des deux parties 32 et 34 de la surface extérieure de la coque est telle que les transitions soient lisses à l'extérieur du boîtier, c'est-à-dire que le surmoulage de matière plastique souple n'engendre pas d'aspérités en creux ou en saillie là où il rejoint des zones de matière plastique dure non recouvertes. De plus, l'ensemble de la surface extérieure présente de préférence une texture lisse.

Par ailleurs, le deuxième matériau 34 est présent également à l'intérieur de la coque, dans certaines zones telles que 70, 80, 90. Il peut servir de coussinet anti-choc pour la carte électronique ou la puce de capteur d'image. Par exemple, les rebords visibles aux figures 1 et 2 pour servir de support aux bords de la carte électronique, peuvent maintenant être réalisées par des portions internes de la matière 34 surmoulée. La zone 90 visible sur la figure 3 peut constituer un tel support d'appui de la carte ; seul un petit élément de zone 90 a été représenté à titre d'exemple, mais on comprendra que la configuration de ces zones peut être très variable. Un rebord de matériau souple 34 peut entourer la presque totalité de la périphérie intérieure du boîtier. Les zones intérieures telles que 80 sont reliées aux zones extérieures telles que 90 par des ponts 85 formés lors du surmoulage à travers des ouvertures du premier matériau, ce qui assure le maintien en place à la fois du matériau extérieur et du matériau intérieur. Des ouvertures sont donc ménagées lors du moulage de la forme de coque en premier matériau. Lors du surmoulage du premier matériau par le deuxième, les ponts 85 s'établissent à travers ces ouvertures.

Le deuxième matériau à consistance de gomme lisse est de préférence coloré pour l'agrément esthétique. Il peut être blanc. La lumière extérieure reste absorbée par le premier matériau 32 qui est de préférence noir.

On obtient ainsi un capteur à la fois ergonomique (confortable en bouche du fait de la consistance agréable au toucher du deuxième matériau et du fait des angles arrondis qu'il présente), résistant mécaniquement du fait de la matière rigide, protégé contre les chocs en ce qui concerne la puce de capteur d'image (protection par les zones intérieures du deuxième matériau). Il n'est pas nécessaire de prévoir des mousses de protection interne de sorte que l'encombrement peut être réduit au minimum. Les blindages électromagnétiques (aluminium, plomb) peuvent être réduits ou supprimés dans le cas de capteurs d'image de type CMOS qui sont moins sensibles aux perturbations électromagnétiques.

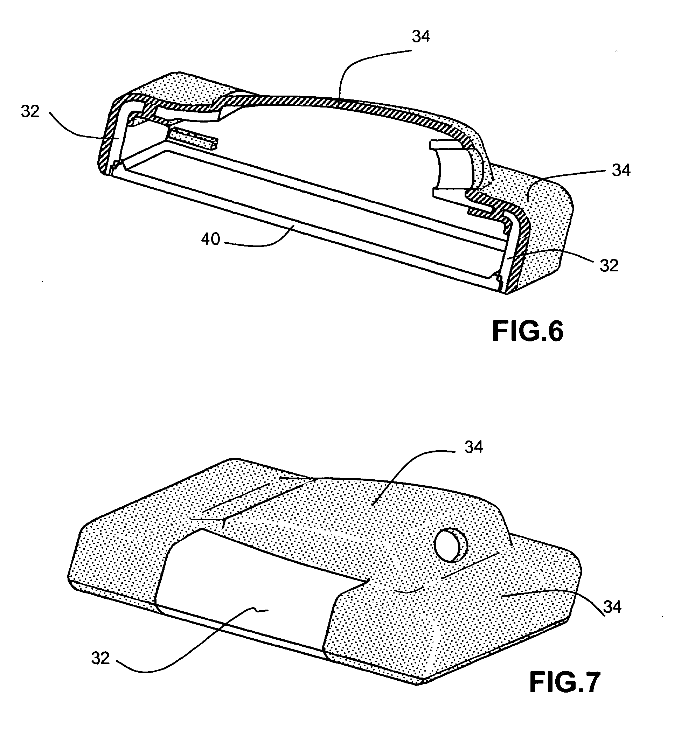

Etant donné que la forme générale du capteur intra oral est généralement parallélépipédique mais présente nécessairement un dôme pour la sortie du câble de liaison avec l'extérieur, on peut prévoir, dans une autre réalisation que ce dôme est réalisé dans le deuxième matériau, non surmoulé sur le premier à cet endroit. Dans cette réalisation, le dôme est plus souple que le reste de la coque, ce qui peut s'avérer particulièrement confortable pour le patient. Les figures 6 et 7 représentent une telle configuration : le dôme est constitué uniquement par de la matière plastique souple qui ne repose pas sur de la matière plastique dure ; il y a cependant continuité de matière plastique souple entre le dôme et d'autres portions de matière plastique souple reposant sur de la matière plastique dure. En d'autres mots, le dôme autoporté est solidaire de portions de matière plastique souple reposant sur de la matière plastique dure, ce qui assure sa fixation au reste du boîtier.

L'épaisseur de couche de matériau souple 34 peut être plus fine sur le dôme que sur les autres parties de la coque.

Dans une variante de réalisation, on peut prévoir qu'une zone de matière plastique souple est spécifiquement prévue pour aider le praticien à positionner le capteur à l'intérieur de la bouche du patient. Cette zone est par exemple située vers le centre de la coque (et elle peut être sur le dôme de sortie du câble) ; la zone a par exemple une forme circulaire et une taille d'environ un centimètre de diamètre, c'est-à-dire une taille correspondant à la surface de l'extrémité d'un doigt ; le praticien sentira cette zone au toucher et saura mieux sentir quelle est la position du capteur dans la bouche.

## Revendications

1. Capteur d'image radiologique dentaire intra-oral comportant un module électronique de prise d'image et un boîtier moulé en matière plastique dure (32), le boîtier comportant localement des surmoulages (34) en une matière plastique plus souple à consistance de gomme lisse recouvrant la matière plastique dure sur des zones situées à l'extérieur du boîtier et correspondant à des portions angulaires saillantes de la matière plastique dure, **caractérisé en ce que** le boîtier comporte également des surmoulages en une matière plastique souple sur des zones situées à l'intérieur du boîtier en des endroits où le module électronique peut venir s'appuyer, les parties surmoulées en matière souple à l'intérieur du boîtier (70, 90) étant reliées à des parties en matière souple à l'extérieur du boîtier par des ponts (85) formés dans des ouvertures ménagées dans les parties en matière dure.

2. Capteur d'image radiologique selon la revendication 1, **caractérisé en ce que** la matière plastique dure (32) est du polyamide.

3. Capteur d'image radiologique selon la revendication 1, **caractérisé en ce que** la matière plastique souple (34) est un copolymère de type SEBS (Styrène-Ethylène-Butylène-Styrène).

4. Capteur d'image radiologique selon l'une des revendications 1 à 3, **caractérisé en ce que** le boîtier comporte un dôme pour une sortie de câble et ce dôme est constitué uniquement de matière plastique souple, ne reposant pas sur de la matière plastique dure mais solidaire d'autres portions de matière plastique souple reposant sur de la matière plastique dure.

5. Capteur d'image radiologique dentaire selon l'une des revendications 1 à 4, **caractérisé en ce que** le boîtier est formé d'une coque (30) et d'une plaque de fond soudée sur la coque, la coque comportant des zones surmoulées en matière plastique souple.

6. Capteur d'image radiologique selon la revendication 5, **caractérisé en ce qu'**une partie de la coque est spécifiquement destinée à former une zone sensible au toucher pour le doigt du praticien afin de l'aider à positionner le capteur dans la bouche du patient, cette zone sensible spécifique étant revêtue de la matière plastique souple à consistance de gomme lisse.

7. Capteur d'image radiologique dentaire selon l'une des revendications 5 et 6, **caractérisé en ce que** la plaque de fond est soudée par ultrasons sur une partie de matière plastique dure de la coque.

## Claims

1. An intra-oral dental radiology image sensor comprising an electronic image acquisition module and a molded casing (32) made of a hard plastic, the casing locally including overmoldings (34) made of a softer plastic having the consistency of smooth rubber covering the hard plastic in areas located on the outside of the casing and corresponding to projecting angular portions of the hard plastic, **characterized in that** the casing also comprises overmoldings in a soft plastic material in areas located inside the casing at places where the electronic module may bear, the overmolding parts in soft material inside the casing (70, 90) being connected to parts in soft material outside the casing by bridges (85) formed in openings formed in the hard material parts.

2. The radiology image sensor as claimed in claim 1, **characterized in that** the hard plastic (32) is a polyamide.

3. The radiology image sensor as claimed in claim 1, **characterized in that** the soft plastic (34) is an SEBS (styrene/ethylene-butylene/styrene) copolymer.

4. The radiology image sensor as claimed in one of claims 1 to 3, **characterized in that** the casing includes a dome through which a cable passes, and this dome consists only of soft plastic, not resting on hard plastic but fastened to other soft plastic portions resting on hard plastic.

5. The dental radiology image sensor as claimed in one of claims 1 to 4, **characterized in that** the casing is formed from a shell (30) and a baseplate welded to the shell, the shell having soft plastic overmolded areas.

6. The dental radiology image sensor as claimed in claim 5, **characterized in that** a portion of the shell is specifically dedicated to form a touch sensitive region for the finger of an operator so as to help positioning the sensor in the patient's mouth, this sensitive region being covered with the soft plastic having a consistentcy of smooth rubber.

7. The dental radiology image sensor as claimed in one of claims 6 and7, **characterized in that** the baseplate is ultrasonically welded to a hard plastic part of the shell.

## Patentansprüche

1. Intraoraler Bildsensor für zahnärztliches Röntgen, der ein elektronisches Bildaufnahmemodul und ein aus einem Duroplast geformtes Gehäuse (32) umfasst, wobei das Gehäuse örtlich mit einem elastischeren Plastikmaterial mit der Konsistenz von glattem Gummi überspritzt (34) ist, das den Duroplast in den Zonen bedeckt, die sich außerhalb des Gehäuses befinden und die vorstehenden Winkelabschnitten des Duroplasts entsprechen, **dadurch gekennzeichnet, dass** das Gehäuse auch Überspritzungen aus einem elastischen Plastikmaterial in Zonen aufweist, die sich im Innern des Gehäuses an Stellen befinden, an denen das elektronische Modul aufliegen kann, wobei die überspritzten Teile aus flexiblem Material im Innern des Gehäuses (70, 90) mit Teilen aus elastischem Material auf der Außenseite des Gehäuses durch Brücken (85) verbunden sind, die in den Öffnungen in den Duroplastteilen ausgebildet sind.

2. Röntgenologischer Bildsensor nach Anspruch 1, **dadurch gekennzeichnet, dass** der Duroplast (32) Polyamid ist.

3. Röntgenologischer Bildsensor nach Anspruch 1, **dadurch gekennzeichnet, dass** das elastische Plastikmaterial (34) ein SEBS-Copolymer (Styrol-Ethylen-Butylen-Styrol) ist.

4. Röntgenologischer Bildsensor nach einem der Ansprüche 1 bis 3, **dadurch gekennzeichnet, dass** das Gehäuse eine Kuppel als Kabelausgang umfasst und diese Kuppel nur aus elastischem Plastikmaterial gebildet ist und nicht auf Duroplast ruht, sondern einstückig mit anderen elastischen Plastikmaterialteilen ausgebildet ist, die auf dem Duroplast ruhen.

5. Zahnröntgenologischer Bildsensor nach einem der Ansprüche 1 bis 4, **dadurch gekennzeichnet, dass** das Gehäuse aus einer Schale (30) und einer an die Schale geschweißten Bodenplatte gebildet ist, wobei die Schale mit elastischem Plastikmaterial überspritzte Zonen umfasst.

6. Röntgenologischer Bildsensor nach Anspruch 5, **dadurch gekennzeichnet, dass** ein Teil der Schale speziell zum Bilden einer Zone bestimmt ist, die für eine Berührung durch den Finger des Arztes empfindlich ist, um ihm beim Positionieren des Sensors im Mund des Patienten zu helfen, wobei diese Zone speziell mit dem elastischen Plastikmaterial mit der Konsistenz von glattem Gummi überzogen ist.

7. Zahnröntgenologischer Bildsensor nach einem der Ansprüche 5 und 6, **dadurch gekennzeichnet, dass** die Bodenplatte an einen Teil des Duroplasts der Schale ultraschallgeschweißt ist.
